# FASCICULE DE BREVET EUROPEEN

(11) **EP 0 544 110 B1**
(45) Date de publication et mention de la délivrance du brevet: **19.07.1995**
(21) Numéro de dépôt: 92118287.9
(22) Date de dépôt: 26.10.1992
(51) Int. Cl.: C07C 35/08, C07C 35/18, C11B 9/00, A61K 7/46

(54) **Alcools tertiaires cycliques et leur utilisation à titre d'ingrédients parfumants**
Tertiäre cyclische Alkohole und ihre Anwendung als Riechstoffbestandteile
Tertiary cyclic alcools and their use as perfume component

(30) Priorité: 25.11.1991 CH 3444/91
(43) Date de publication de la demande: 02.06.1993
(73) Titulaire: FIRMENICH SA, CH-1211 Genève 8 (CH)
(72) Inventeur: Decorzant, René, CH-1213 Onex (CH); Naef, Ferdinand, CH-1227 Carouge (CH)
(74) Mandataire: Salvadori, Giuseppe

(56) Documents cités:
- TETRAHEDRON LETTERS vol. 25, no. 9, 1984, OXFORD GB pages 907 - 910 R.H. MITCHELL ET AL 'CYCLOPHANES WITH LARGE INTERNAL SUBSTITUENTS.THE SYNTHESIS AND CONFORMATIONAL BEHAVIOUR OF 2,11-DITHIA[3,3]METACYCLOPHANES AND A [2,2]METACYCLOPHANE WITH tert-BUTYL SUBSTITUENTS.' COMPOUND 4
- 'CHEMICAL ABSTRACTS SERVICE.REGISTRY HANDBOOK. NUMBER SECTION - 1986 SUPPLEMENT' 1986 , THE AMERICAN CHEMICAL SOCIETY , COLUMBUS, OHIO ,US RN=105875-60-3

## Description

La présente invention a trait au domaine de la parfumerie. Elle concerne, plus particulièrement des composés nouveaux de formule 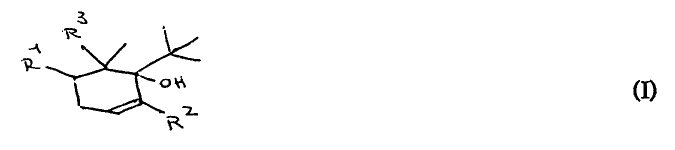
dans laquelle les symboles R¹ et R³ représentent chacun un atome d'hydrogène ou un radical méthyle et le symbole R² représente un radical alkyle inférieur. Par radical alkyle inférieur on entend ici un radical alkyle ayant de 1 à 6, de préférence de 1 à 3, atomes de carbone.

Nous avons découvert que ces composés possèdent des propriétés odorantes très utiles et qu'ils peuvent, de ce fait, être employés pour la préparation de compositions parfumantes et articles parfumés, auxquels ils confèrent des notes rappelant l'odeur de l'essence de patchouli.

Parmi les composés de formule (I) on peut citer à titre préférentiel le 1-tert-butyl-2,6,6-triméthyl-2-cyclohexén-1-ol. Ce composé possède une note odorante très intéressante s'apparentant à l'odeur du patchouli avec un côté camphré, boisé-poudré et damasconé. Il s'agit d'une note évoquant les sous-bois et la partie résineuse du patchouli, tout en ayant un caractère moins terreux.

L'odeur de ce composé est d'autant plus utile, que l'on sait que ce type de note est extrêmement rare parmi les produits d'origine synthétique. En plus, bien que la note odorante de type patchouli soit une des plus importantes en parfumerie, les composants synthétiques de l'essence de patchouli, tels que le patchoulol ou le patchoulénol, sont tellement difficiles à préparer qu'ils ne s'avèrent pas compétitifs au point de vue prix. Si l'on considère encore les problèmes liés aux fluctuations inhérentes à la production de l'essence d'origine naturelle, on comprendra davantage l'importance que peut revêtir la découverte d'un produit d'origine synthétique possédant des propriétés odorantes capables de reproduire celles des produits d'origine naturelle. La présente invention apporte donc une solution nouvelle à ce problème.

Les propriétés odorantes des autres composés (I) selon l'invention sont présentées plus loin lors de la description de leur préparation. En plus de leur note commune de type patchouli, chacun de ces composés possède d'autres caractères olfactifs distinctifs, de sorte que l'utilisation de l'ensemble de ces composés en parfumerie permet un choix varié de nuances olfactives, et ainsi d'applications, dans le domaine des compositions à odeur de type patchouli.

De par leurs propriérés odorantes, les composés (I) selon l'invention se prêtent aussi bien aux applications en parfumerie fine, qu'au parfumage de produits fonctionnels. C'est ainsi qu'ils peuvent être employés de façon avantageuse pour la préparation de compositions et bases parfumantes, parfums et eaux de toilette, mais aussi dans le parfumage d'articles divers tels les savons, les gels de bain ou douche, les shampoings et autres produits d'hygiène capillaire, les désodorisants corporels ou d'air ambiant et les préparations cosmétiques. Ils se prêtent également au parfumage de détergents ou adoucissants textiles, ou encore de produits d'entretien.

Dans ces applications, les composés (I) peuvent être utilisés seuls ou, comme il est plus courant en parfumerie, mélangés à d'autres ingrédients parfumants, solvants ou adjuvants usuels. Une énumération plus détaillée de la nature de ces co-ingrédients est ici superflue, l'homme du métier étant à même de les choisir en fonction de l'effet olfactif qu'il désire obtenir. Pour ce faire il peut notamment avoir recours à des oeuvres de référence telles que le livre de S. Arctander, Perfume and Flavor Chemicals, Montclair, N.J., USA (1976). L'effet olfactif désiré conditionne également le choix des proportions dans lesquelles les composés de l'invention peuvent être utilisés pour les applications susmentionnées, proportions qui dépendent également de la nature des autres ingrédients présents dans une composition donnée.

Bien que ces proportions puissent ainsi varier dans une gamme de valeurs très étendue, on peut citer, à titre d'exemple, des concentrations de l'ordre de 5 à 10%, voire même 20% en poids, par rapport au poids de la composition parfumante dans laquelle ils sont incorporés. Des concentrations bien inférieures à ces valeurs seront normalement utilisées lorsque les composés (I) seront utilisés pour le parfumage des articles divers mentionnés plus haut.

Les composés de l'invention sont préparés à partir de cétones de formule 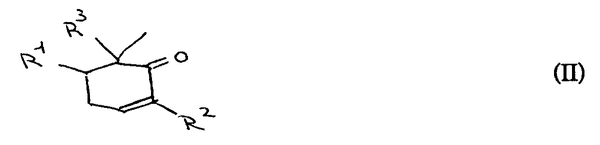
dans laquelle les symboles R¹ à R³ ont le sens indiqué à la formule (I), en faisant réagir ces cétones avec du tert-butyl-lithium, dans un solvant organique inerte. Alternativement, du tert-butyl-magnesium peut être utilisé dans des conditions similaires, ou bien dans les conditions usuelles de la réaction dite de Barbier [voir, par ex., J. March, Advanced Organic Chemistry, 3^{ème} ed., page 817, ed. Wiley Interscience, NY (1985)]. Les cétones de départ de formule (II) sont des produits qui peuvent soit être obtenus sur le marché, soit être préparés par des méthodes décrites dans l'art antérieur.

La méthode générale de préparation des composés (I) est décrite ci-après pour le cas particulier du

### 1-tert-butyl-2,6,6-triméthyl-2-cyclohexén-1-ol

Dans un ballon à 4 cols de 350 ml, muni d'un agitateur magnétique, sous atmosphère d'argon, ont été placés 13,8 g (0,1 mole) de 2,6,6-triméthyl-2-cyclohexén-1-one (voir DE-PS 25 47 223) en solution dans 100 ml d'éther diéthylique.
100 Ml (0,14 mole) de tert-butyl-lithium, 1,4 N en solution dans le pentane (Fluka), ont été ajoutés goutte à goutte à -40°C. L'agitation a été maintenue encore 3 h en laissant revenir la température à 20°C. Le mélange réactionnel a ensuite été versé sur de la glace (100 ml), puis extrait à l'éther (2 x 100 ml), les phases éthérées ayant été lavées successivement avec HCl 1 N (50 ml), NaHCO₃ (50 ml) et eau saturée de sel (2 x 50 ml), séchées (MgSO₄) et concentrées à pression réduite.
On a obtenu 18,8 g de concentrat qui a été distillé sur colonne Vigreux (10 cm) pour fournir 3 fractions comme suit (pression : 18x10² Pa)

| | | |
|---|---|---|
| Fraction 1 | 80-84°C (bain → 100°C) | 0,3 g de produit de départ |
| Fraction 2 | 84-110°C (100 → 126°C) | 0,4 g |
| Fraction 3 | 110-112°C (126 → 134°C) | 13,5 g d'un mélange contenant |
| | | 86% du produit désiré et |
| | | 10% d'un produit secondaire; |

et 1,4 g de résidus.
La troisième fraction de distillation a ensuite été chromatographiée sur acide silicique, Merck, 0,2-0,063, ∼270 g (20 fois), en utilisant comme éluant un mélange de cyclohexan-éther 98:2, pour fournir une fraction enrichie en le produit désiré, laquelle, après distillation au four à boules (130-150°C/18x10² Pa) a fourni 7,3 g de 1-tert-butyl-2,6,6-triméthyl-2-cyclohexén-1-ol pur à 97% (rend. 37%).
Données analytiques :
RMN(¹H, 360MHz, CDCl₃) : 0,94(s, 3H) ; 1,09(s, 9H) ; 1,12(s, 3H) ; 1,35(m, 1H) ; 1,80(d finement divisé, J=2Hz, 3H) ; 2,07(m, 2H) ; 2,14(m, 1H) δ ppm.
RMN(¹³C, 90,5MHz, CDCl₃) : 137,6(s) ; 124,5(d) ; 81,2(s) ; 42,8(s) ; 41,1(s) ; 34,4(t) ; 29,4(q) ; 28,6(q) ; 27,6(q) ; 22,6(t) ; 22,1(q) δ ppm.
SM : 196(M⁺, 0), 178(1), 163(1), 139(29), 121(14), 107(7), 95(48), 83(19), 67(6), 55(15), 43(100).
Odeur : décrite plus haut.
Suivant un procédé analogue à celui décrit à-dessus mais en employant comme produit de départ la cétone (II) appropriée, les produits suivants ont été également préparés :

### 1-tert-butyl-2-éthyl-6,6-diméthyl-2-cyclohexén-1-ol

Préparé à partir de la 2-éthyl-6,6-diméthyl-2-cyclohexén-1-one (voir H. Hopf et N. Krause, Liebigs Ann. Chem. 1987, 943).
Données analytiques :
RMN(¹H, 360MHz, CDCl₃) : 0,95(s, 3H) ; 1,03(t, J=7Hz, 3H) ; 1,06(s, 9H) ; 1,12(s, 3H) ; 1,36(m, 1H) ; 1,99-2,24(m, 5H) ; 5,48(s large, 1H) δ ppm.
SM : 210(M⁺, 0), 192(1), 177(1), 153(46), 135(9), 121(4), 109(50), 97(21), 81(7), 69(15), 57(33), 43(100).
Odeur : boisée, légèrement ambrée, cèdre, patchouli, piquante-irritante, acide.

### 1-tert-butyl-2,5,6-triméthyl-2-cyclohexén-1-ol

Préparé à partir de la 2,5,6-triméthyl-2-cyclohexén-1-one (voir, par exemple US Publ. Pat. App. B 501,128). Le produit obtenu était un mélange de 3 stéréoisomères (∼20% ∼60% -20%).
Données analytiques :
RMN(¹H, 360MHz, CDCl₃) : mélange de 3 isomères : 0,79(d, J=7Hz) ; 0,90(d, J=7Hz) ; 0,97(s) ; 1,02(d, J=7Hz) ; 1,04(s) ; 1,05(s) ; 1,05-1,25(m) ; 1,39-1,54(m) ; 1,67-2,63(m) ; 1,79(s large) ; 1,83(s large) ; 5,42(s large) ; 5,47(s large) ; 5,83(d, J=8Hz) δ ppm.

### Isomère 1

SM : 196(M⁺, 0), 178(5), 163(5), 149(1), 139(97), 121(41), 105(26), 95(53), 79(16), 69(19), 57(60), 43(100).

### Isomère 2

SM : 196(M⁺, 1), 178(10), 163(10), 149(2), 139(100), 121(52), 107(25), 95(47), 83(23), 69(31), 57(64), 43(73).

### Isomère 3

SM : 196(M⁺, 1), 178(16), 163(9), 149(1), 139(87), 121(51), 107(29), 95(43), 83(25), 69(54), 57(100), 43(73).
Odeur : boisée, légèrement ambrée, cèdre, patchouli, acide, piquanteirritante.

### cis-1-tert-butyl-2,5,6,6-tétraméthyl-2-cyclohexén-1-ol

Préparé à partir de la 2,5,6,6-tétraméthyl-2-cyclohexén-1-one (voir, par exemple DE-OS 24 44 585).
Données analytiques :
RMN(¹H, 360MHz, CDCl₃) : 0,78(s, 3H) ; 0,86(d, J=7Hz, 3H) ; 1,09(s, 9H) ; 1,50-1,63(m, 1H) ; 1,79(s large, 3H) ; 2,09-2,21(m, 1H) ; 2,33-2,49(m, 1H) ; 5,47(s large, 1H) δ ppm.
RMN(¹³C, 90,5MHz, CDCl₃) : 137,5(s) ; 124,9(d) ; 82,0(s) ; 44,6(s) ; 43,0(s) ; 33,3(d+t) ; 30,2(3xq) ; 24,8(q) ; 22,3(q) ; 19,8(q) ; 16,9(q) δ ppm.
SM : 210(M⁺, 0), 192(2), 177(3), 163(1), 153(51), 135(25), 121(17), 109(48), 91(17), 82(61), 69(11), 57(23), 43(100).
Odeur : boisée, patchouli, moisi.

L'invention sera maintenant décrite plus en détail à l'aide des exemples suivants.

### Exemple 1

### Composition parfumante pour shampoing

On a préparé une composition parfumante de base, destinée au parfumage d'un shampoing, par mélange des ingrédients suivants :

| Ingrédients | Parties en poids |
|---|---|
| Alcool phényléthylique | 130 |
| Ethylène brassylate | 20 |
| Essence d'aspic | 80 |
| Essence de géraniol synthétique | 80 |
| Essence de bergamote synthétique | 80 |
| Acétate de linalyle | 60 |
| Essence de géranium synthétique | 60 |
| Mousse de chêne absolue à 10%* | 60 |
| Essence de patchouli | 40 |
| Labdanum résinoïde à 10%* | 40 |
| Acétate de p-tert-butylcyclohexyle | 30 |
| Galaxolide ® 50 ¹⁾ | 50 |
| Coumarine | 30 |
| Linalol | 60 |
| Terpinéol | 30 |
| Méthylisoeugénol | 30 |
| Essence de romarin | 20 |
| Essence de galbanum à 10%* | 20 |
| Sandela ® ²⁾ | 20 |
| Civette synthétique à 10%* | 20 |
| Essence de neroli synthétique | 15 |
| Lavandin absolu | 10 |
| Lilial ® ³⁾ | 10 |
| Essence de myrte | 5 |
| Total | 1̅0̅0̅0̅ |

| | |
|---|---|
| * dans le dipropylèneglycol (DIPG) | |
| 1) 1,3,4,6,7,8-hexahydro-4,6,6,7,8,8-hexaméthyl-cyclopenta[g]isochromène ; origine : IFF Inc., USA | |
| 2) 3-(isocamphyl-5)-cyclohexan-1-ol ; origine : L. Givaudan, Vernier, Suisse | |
| 3) p-tert-butyl-α-méthylhydrocinnamaldéhyde; origine : L.Givaudan, Vernier, Suisse. | |

Lorsqu'on a remplacé dans cette composition de base de type boisé, herbacé, floral, les 4% en poids d'essence de patchouli par la même quantité de 1-tert-butyl-2,6,6-triméthyl-2-cyclohexén-1-ol, on a obtenu une composition nouvelle avec un net caractère patchouli mais moins terreux et plus boisé que le caractère olfactif imparti par l'essence d'origine naturelle.

### Exemple 2

### Composition parfumante de type féminin

On a préparé une composition de base destinée à un parfum de type féminin, par mélange des ingrédients suivants :

| Ingrédients | Parties en poids |
|---|---|
| Essence de coriandre | 100 |
| Acétate de benzyle | 100 |
| Acétate de styrallyle | 80 |
| Acétate de vétyveryle | 1150 |
| Aldéhyde undécylénique à 10%* | 150 |
| Aldéhyde hexylcinnamique | 100 |
| Citronellol | 250 |
| Dioxycarbinol | 450 |
| Exaltolide ® ¹⁾ | 200 |
| Géraniol | 300 |
| Iralia ® ²⁾ | 50 |
| Jasmin absolu | 150 |
| Labdanum ciste à 10%* | 200 |
| Linalol | 200 |
| Lyral ® ³⁾ | 700 |
| Mousse de chêne absolue Dalma à 50%* | 200 |
| Hedione ® ⁴⁾ | 3000 |
| Octylcarbonate de méthyle | 200 |
| Oxyde de rose à 10%* | 25 |
| Alcool phényléthylique | 450 |
| Rosinol crist. | 50 |
| Salicylate d'amyle | 75 |
| Salicylate de benzyle | 200 |
| Vanilline à 1%* | 220 |
| α-Ionone | 200 |
| Essence d'ylang extra | 200 |
| Essence de patchouli | 1000 |
| Total | 1̅0̅0̅0̅0̅ |

| | |
|---|---|
| * dans le dipropylèneglycol (DIPG) | |
| 1) pentadécanolide; origine : Firmenich SA, Genève, Suisse | |
| 2) méthylionone; origine : Firmenich SA, Genève, Suisse | |
| 3) 4-(4-hydroxy-4-méthylpentyl)-3-cyclohexène-1-carboxaldéhyde; origine : IFF Inc., USA | |
| 4) dihydrojasmonate de méthyle; origine : Firmenich SA, Genève, Suisse. | |

Lorsqu'on a remplacé dans cette composition de base de type boisé, aromatique, floral, les 10% en poids d'essence de patchouli par 5% en poids de 1-tert-butyl-2,6,6,-triméthyl-2-cyclohexén-1-ol, on a obtenu une composition nouvelle dont le caractère patchouli s'est trouvé renforcé et doublé d'une note boisée racineuse très naturelle. En plus, la composition nouvelle avait beaucoup plus d'impact que la composition de base.

## Revendications

1. Composés de formule 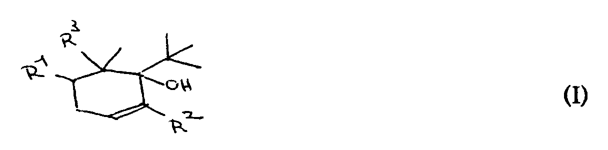 dans laquelle les symboles R¹ et R³ représentent chacun un atome d'hydrogène ou un radical méthyle et le symbole R² représente un radical alkyle inférieur.

2. 1-tert-Butyl-2,6,6-triméthyl-2-cyclohexén-1-ol.

3. Utilisation d'un composé selon la revendication 1 à titre d'ingrédient parfumant.

4. Utilisation du 1-tert-butyl-2,6,6-triméthyl-2-cyclohexén-1-ol à titre d'ingrédient parfumant.

5. Composition parfumante ou article parfumé contenant à titre d'ingrédient parfumant un composé selon la revendication 1.

6. Composition parfumante ou article parfumé contenant à titre d'ingrédient parfumant le 1-tert-butyl-2,6,6-triméthyl-2-cyclohexén-1-ol.

7. Article parfumé selon la revendication 5 ou 6, sous forme d'un parfum ou d'une eau de toilette, d'un savon, d'un gel de douche ou bain, d'un shampoing ou autre produit d'hygiène capillaire, d'un désodorisant corporel ou d'air ambiant, d'une préparation cosmétique, d'un détergent ou adoucissant textile ou d'un produit d'entretien.

## Claims

1. Compounds of formula 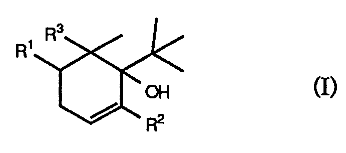 wherein symbols R¹ and R³ represent each a hydrogen atom or a methyl radical and symbol R² represents a lower alkyl radical.

2. 1-tert-Butyl-2,6,6-trimethyl-2-cyclohexen-1-ol.

3. Use of a compound according to claim 1 as a perfuming ingredient.

4. Use of 1-tert-butyl-2,6,6-trimethyl-2-cyclohexen-1-ol as a perfuming ingredient.

5. Perfuming composition or perfumed article containing as a perfuming ingredient a compound according to claim 1.

6. Perfuming composition or perfumed article containing as a perfuming ingredient 1-tert-butyl-2,6,6-trimethyl-2-cyclohexen-1-ol.

7. Perfumed article according to claim 5 or 6, in the form of a perfume or cologne, a soap, a bath or shower gel, a shampoo or other hair-care product, a body or air deodorant, a cosmetic preparation, a detergent or a fabric softener, or a household product.

## Patentansprüche

1. Verbindungen der Formel 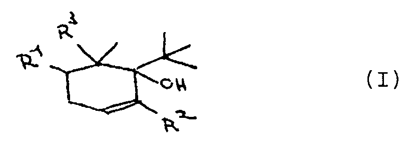 worin die Symbole R¹ und R³ jeweils für ein Wasserstoffatom oder einen Methylrest stehen und das Symbol R² einen niederen Alkylrest bedeutet.

2. 1-tert-Butyl-2,6,6-trimethyl-2-cyclohexen-1-ol.

3. Verwendung einer Verbindung gemäss Patentanspruch 1 als Riechstoffbestandteil.

4. Verwendung des 1-tert-Butyl-2,6,6-trimethyl-2-cyclohexen-1-ols als Riechstoffbestandteil.

5. Riechstoffkomposition oder parfümierter Artikel, enthaltend als Riechstoffbestandteil eine Verbindung gemäss Patentanspruch 1.

6. Riechstoffkomposition oder parfümierter Artikel, als Riechstoffbestandteil enthaltend 1-tert-Butyl-2,6,6-trimethyl-2-cyclohexen-1-ol.

7. Parfümierter Artikel gemäss Patentanspruch 5 oder 6 in Form eines Parfüms oder eines Toilettwassers, einer Seife, eines Douche- oder Badegels, eines Shampoos oder eines anderen Produktes der Haarpflege, eines Körperdesodorants oder eines Raumluftverbesserers, einer kosmetischen Zusammensetzung, eines Detergenz oder eines Textilauffrischungsmittels oder eines Pflegeproduktes.
